(19) Europäisches Patentamt
European Patent Office
Office européen des brevets

(11) EP 3 023 109 A1

(12) EUROPEAN PATENT APPLICATION
published in accordance with Art. 153(4) EPC

(43) Date of publication:
25.05.2016 Bulletin 2016/21

(51) Int Cl.:
A61K 47/36 (2006.01)          A61K 9/20 (2006.01)
A61K 31/445 (2006.01)         A61K 47/02 (2006.01)
A61K 47/32 (2006.01)          A61P 3/10 (2006.01)
A61P 43/00 (2006.01)

(21) Application number: 14827092.9

(22) Date of filing: 17.07.2014

(86) International application number:
PCT/JP2014/069024

(87) International publication number:
WO 2015/008825 (22.01.2015 Gazette 2015/03)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 19.07.2013 JP 2013150410

(71) Applicant: Sanwa Kagaku Kenkyusho Co., Ltd
Nagoya-shi
Aichi 461-8631 (JP)

(72) Inventors:
• KONDO, Masahiro
Nagoya-shi
Aichi 461-8631 (JP)
• OCHIAI, Naoya
Nagoya-shi
Aichi 461-8631 (JP)
• SAKAKURA, Yuki
Nagoya-shi
Aichi 461-8631 (JP)

(74) Representative: Raynor, Stuart Andrew
J A Kemp
14 South Square
Gray's Inn
London WC1R 5JJ (GB)

(54) ORALLY DISINTEGRATING TABLET

(57) Disclosed is an orally disintegrating tablet including: (a) an active ingredient; (b) a starch with an amylose content of 20% by mass to 30% by mass and a degree of gelatinization of less than 10%; and (c) at least one inorganic excipient selected from the group consisting of magnesium silicate, calcium silicate, and synthetic hydrotalcite. The orally disintegrating tablet is a single-layer tablet or a dry-coated tablet having an inner core and an outer layer. The orally disintegrating tablet as a single-layer tablet or the outer layer of the orally disintegrating tablet as a dry-coated tablet has a crystalline cellulose content of 0% by mass to 5% by mass.

Fig.1

## Description

## Technical Field

The present invention relates to orally disintegrating tablets.

## Background Art

Among the dosage forms used at present, tablets have a particularly high level of handleability and easiness of taking. However, tablets are not always good dosage forms for patients with a swallowing function problem, such as elderly people and children. Therefore, orally disintegrating tablets have been developed as dosage forms for solving this problem, which contributes to improvement of medication compliance of such patients.

In some cases, orally disintegrating tablets are generally designed to have a very low density structure so that saliva can quickly enter their inside to achieve rapid disintegration of them in the oral cavity (their properties). However, such tablet preparations are very brittle and can cause problems such as cracking and chipping during transportation or handling for taking.

In recent years, the brittleness of such tablets has been compensated for in the design of orally disintegrating tablets. Patent Literature 1 describes one of such orally disintegrating tablets, which is a rapidly disintegrating preparation obtained by a process including forming a wet powder into a tablet using a tableting machine having a special mechanism; and then drying the wet tablet in a dryer.

Some orally disintegrating tablets are produced without using the special manufacturing equipment mentioned above. Patent Literature 2 describes an example of such orally disintegrating tablets, which are orally rapidly disintegrating tablets including an active ingredient, crystalline cellulose, and an inorganic excipient, in which the crystalline cellulose and the inorganic excipient are present in a predetermined ratio. Patent Literatures 3 to 7 describe orally disintegrating tablets obtained by granulating a sugar alcohol or the like with a water-insoluble hydrophilic excipient such as starch and subjecting the granules to compression molding. Patent Literature 8 describes an orally disintegrating tablet containing an active ingredient and granulated particles having an inorganic material and a disintegrator uniformly dispersed in composite particles of two or more saccharides. Patent Literatures 9 and 10 describe an orally disintegrating tablet containing (1) an active ingredient, (2) an inorganic excipient, (3) crystalline cellulose, and (4) a natural starch. Patent Literature 11 describes an orally disintegrating tablet including (1) a sugar alcohol or the like, (2) starch or the like, and (3) a lubricant. Patent Literature 12 describes an orally disintegrating tablet including (1) an active ingredient, (2) 15% by mass or less of calcium silicate, (3) a disintegrator, and (4) a diluent such as a sugar alcohol or crystalline cellulose. Patent Literature 13 describes a directly compressible composite including a water-soluble excipient such as a sugar alcohol and calcium silicate. Patent Literature 14 describes an orally disintegrating tablet including (1) an active ingredient, (2) a water-insoluble moiety such as crystalline cellulose, (3) a surfactant, and (4) a disintegrator. Patent Literature 15 describes an orally disintegrating tablet including (1) an active ingredient, (2) a superdisintegrant such as crospovidone, (3) a dispersing agent including calcium silicate, and (4) a dispersing agent such as a sugar alcohol.

These orally disintegrating tablets prepared without using the special manufacturing equipment often contain crystalline cellulose (water-insoluble cellulose) or a sugar alcohol in order to have good physical properties. They also tend to contain increased amounts of a disintegrator and an excipient such as a binder in order to maintain a certain level of oral disintegrability and strength.

Prior Art Document

Patent Document

Patent Document 1: Japanese Patent No. 4162405
Patent Document 2: Japanese Patent No. 3996626
Patent Document 3: Japanese Patent Application Laid-Open Publication No. 2010-155865
Patent Document 4: Japanese Patent No. 4446177
Patent Document 5: Japanese Patent No. 5062871
Patent Document 6: Japanese Patent No. 4551627
Patent Document 7: Japanese Patent No. 5062872
Patent Document 8: Japanese Patent Application National Publication No.2011-513194
Patent Document 9: International Publication No. WO 2010/134574
Patent Document 10: International Publication No. WO 2009/066773 A

Patent Document 11: Japanese Patent No. 4802436
Patent Document 12: Japanese Patent Application National Publication No.2010-540588
Patent Document 13: Japanese Patent Application National Publication No.2009-532343
Patent Document 14: Japanese Patent Application National Publication No.2011-506279
Patent Document 15: Japanese Patent Application National Publication (Laid-Open) No.2005-507397

## Summary of Invention

### Technical Problem

As mentioned above, conventional orally disintegrating tablets often have a higher content of excipients other than active ingredients so that they can have desired properties such as a certain level of both disintegrability and strength. However, as the content of active ingredients increases, the balance between the strength and disintegrability of tablets tends to decrease. In fact, orally disintegrating tablets (267 items) already launched on the market in Japan have an average active ingredient content in a tablet as low as about 7.2% by mass. In an aspect, therefore, it is an object of the present invention to provide an orally disintegrating tablet that is less likely to be affected by the physical properties and content of active ingredients and the like and has both rapid disintegrability in the oral cavity and high strength.

### Solution to Problem

In view of the above problems, the inventors have made intensive studies and, as a result, have found that crystalline cellulose, generally used in conventional orally disintegrating tablets, is more likely to be affected by the physical properties and content of active ingredients, is less likely to impart a desired level of disintegrability and strength to tablets, and can rather have an adverse effect on the physical properties of orally disintegrating tablets. Thus, the inventors have come up with an orally disintegrating tablet substantially free of crystalline cellulose and have focused on starch as an excipient for ensuring rapid disintegrability in the oral cavity. In general, starch not having undergone a pharmaceutical process does not have very good compression moldability. Therefore, it is known that as the content of starch increases, abrasion and capping of tablets tend to increase (Kaitei Iyakuhin Tenkabutsu Handbook (Revised Handbook of Pharmaceutical Additives), YAKUJI NIPPO LIMITED, pp. 603-610, 2007). Nevertheless, the inventors have found that the use of a combination of a specific starch and a specific inorganic excipient makes it possible to overcome the problem with the use of starch not having undergone a pharmaceutical process and also makes it possible to provide an orally disintegrating tablet that has both rapid disintegrability in the oral cavity and high strength even when having a high active ingredient content. The inventors have completed the present invention based on the findings. The inventors have further found that like crystalline cellulose mentioned above, sugar alcohols, which are often used in conventional orally disintegrating tablets, can also have an adverse effect on the physical properties of orally disintegrating tablets.
That is, the main composition of the present invention is as follows.

<1> An orally disintegrating tablet comprising: (a) an active ingredient; (b) a starch with an amylose content of 20% by mass to 30% by mass and a degree of gelatinization of less than 10%; and (c) at least one inorganic excipient selected from the group consisting of magnesium silicate, calcium silicate, and synthetic hydrotalcite, the orally disintegrating tablet being a single-layer tablet in which the active ingredient (a), the starch (b), and the inorganic excipient (c) are dispersed and mixed over the whole of the orally disintegrating tablet, the orally disintegrating tablet having a crystalline cellulose content of 0% by mass to 5% by mass based on the total mass of the orally disintegrating tablet.
<2> The orally disintegrating tablet according to <1>, which has a sugar alcohol content of 0% by mass to 15% by mass based on the total mass of the orally disintegrating tablet.
<3> An orally disintegrating tablet comprising: (a) an active ingredient; (b) a starch with an amylose content of 20% by mass to 30% by mass and a degree of gelatinization of less than 10%; and (c) at least one inorganic excipient selected from the group consisting of magnesium silicate, calcium silicate, and synthetic hydrotalcite, the orally disintegrating tablet being a dry-coated tablet having an inner core and an outer layer, wherein the inner core contains the active ingredient (a), and the outer layer contains the starch (b) and the inorganic excipient (c) and has a crystalline cellulose content of 0% by mass to 5% by mass based on the total mass of the outer layer.
<4> The orally disintegrating tablet according to <3>, wherein the outer layer has a sugar alcohol content of 0% by mass to 15% by mass based on the total mass of the outer layer.
<5> The orally disintegrating tablet according to any one of <1> to <4>, which has a total content of the active ingredient (a), the starch (b), and the inorganic excipient (c) of at least 80% by mass based on the total mass of the orally disintegrating tablet (Hereinafter, it may be called to as "the preparation").

<6> The orally disintegrating tablet according to any one of <3> to <5>, which has a content of the active ingredient (a) of 0.1% by mass to 60% by mass based on the total mass of the preparation, has a content of the starch (b) of 20% by mass to 96% by mass (or 20% by mass to 95% by mass) based on the total mass of the preparation, and has a content of the inorganic excipient (c) of 3% by mass to 60% by mass (or 4% by mass to 45% by mass) based on the total mass of the preparation.

<7> The orally disintegrating tablet according to <6>, which has a content of the active ingredient (a) of 2.5% by mass to 55% by mass (or 20% by mass to 55% by mass) based on the total mass of the preparation.

<8> The orally disintegrating tablet according to <6>, which has a content of the starch (b) of 25% by mass to 85% by mass (or 25% by mass to 70% by mass) based on the total mass of the preparation.

<9> The orally disintegrating tablet according to <6>, which has a content of the inorganic excipient (c) of 4% by mass to 45% by mass (or 8% by mass to 30% by mass) based on the total mass of the preparation.

<10> The orally disintegrating tablet according to any one of <1> to <9>, wherein the starch (b) comprises at least one selected from the group consisting of corn starch, potato starch, and wheat starch, or wherein the starch (b) is at least one selected from above group.

<11> The orally disintegrating tablet according to any one of <1> to <10>, further comprising (d) crospovidone.

<12> The orally disintegrating tablet according to <11>, which has a content of the crospovidone (d) of 0.1% by mass to 20% by mass based on the total mass of the preparation.

<13> The orally disintegrating tablet according to any one of <1> to <12>, wherein the active ingredient (a) comprises a water-soluble active ingredient, or wherein the active ingredient (a) is a water-soluble active ingredient.

<14> The orally disintegrating tablet according to <13>, wherein the water-soluble active ingredient comprises an $\alpha$-glucosidase inhibitor, or wherein the water-soluble active ingredient is an $\alpha$-glucosidase inhibitor.

<15> The orally disintegrating tablet according to <14>, wherein the $\alpha$-glucosidase inhibitor comprises miglitol, or wherein the $\alpha$-glucosidase inhibitor is miglitol.

**Advantageous Effects of Invention**

The present invention makes it possible to provide an orally disintegrating tablet that is less likely to be affected by the physical properties or content of active ingredients and has both rapid disintegrability in the oral cavity and high strength. In particular, the present invention makes it possible to provide a superior orally disintegrating tablet that can have the desired physical properties mentioned above even when containing a water-soluble active ingredient and even when the water-soluble active ingredient content is high.

**Brief Description of Drawings**

Fig. 1 is a cross-sectional view showing an embodiment of an orally disintegrating tablet (single-layer tablet); and
Fig. 2 is a cross-sectional view showing an embodiment of an orally disintegrating tablet (dry-coated tablet).

**Description of Embodiments**

Hereinafter, embodiments of the present invention will be described in more detail. It will be understood that the embodiments below are not intended to limit the present invention.

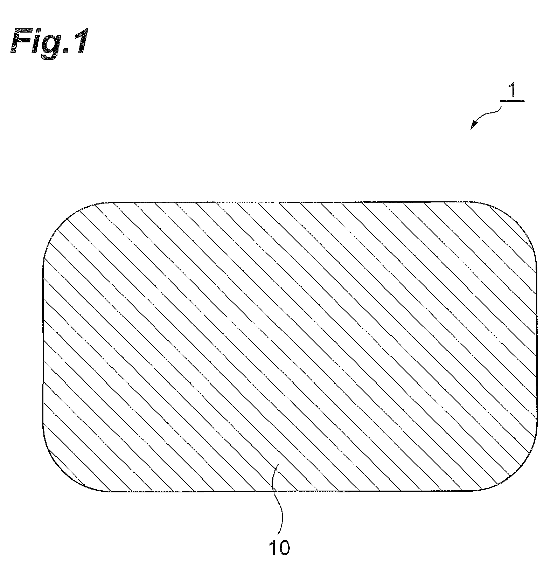

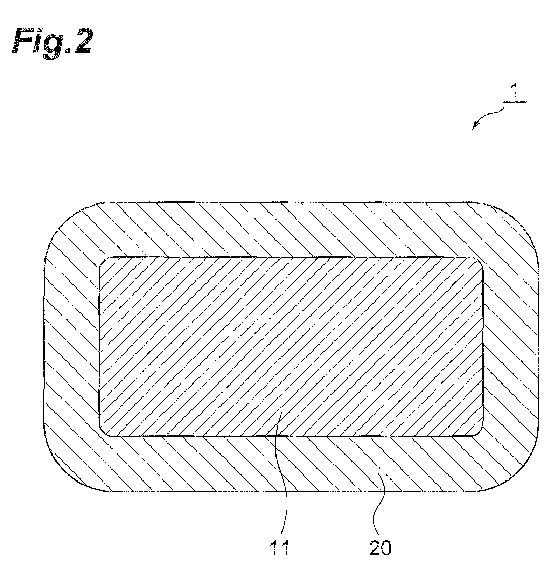

The orally disintegrating tablet according to the embodiment contains a specific starch and a specific inorganic excipient as described below together with an active ingredient and the like. Figs. 1 and 2 are cross-sectional views each showing an embodiment of the orally disintegrating tablet. The orally disintegrating tablet 1 of Fig. 1 is a single-layer tablet including only a single mixture layer 10 in which the active ingredient, the starch, and the inorganic excipient are dispersed and mixed. In the case of this form, the content of each ingredient in the whole of the orally disintegrating tablet (preparation) 1 coincides with the content of each ingredient in the mixture layer 10. The orally disintegrating tablet 1 of Fig. 2 includes an inner core 11 and an outer layer 20 with which the inner core 11 is coated. In general, the inner core 11 principally contains the active ingredient, but the outer layer 20 may contain a part of the active ingredient.

According to the embodiment, the orally disintegrating tablet 1 (mixture layer 10) or the outer layer 20 is substantially free of crystalline cellulose (or water-insoluble cellulose), which is generally used in conventional orally disintegrating tablets. Specifically, the content of crystalline cellulose is 0% by mass to 5% by mass based on the total mass of the preparation or the outer layer. In other words, the content of crystalline cellulose in a mixture containing the specific starch and the specific inorganic excipient described below is 0% by mass to 5% by mass based on the mass of the mixture (the mixture layer 10 or the outer layer 20).

The specific starch (b) that may be contained in the orally disintegrating tablet according to the embodiment has an amylose content of 20% by mass to 30% by mass as measured by an iodine colorimetric method and also has a degree of gelatinization of less than 10% as measured by a glucoamylase method. This type of starch is advantageous in that it is less soluble in water. The orally disintegrating tablet containing the starch with an amylose content of 20% by mass to 30% by mass tends to rapidly disintegrate in the oral cavity. Orally disintegrating tablets containing starch with a degree of gelatinization of 10% or more may have difficulty in having good disintegrability. Examples of the starch with an amylose content of 20% by mass to 30% by mass and a degree of gelatinization of less than 10% include corn starch, potato starch, and wheat starch. In view of the physical properties of the tablet, the starch to be used may be selected from corn starch and potato starch or may be corn starch. These starches may be used singly or in combination of two or more.

The content of the starch may be 20% by mass to 96% by mass, 25% by mass to 85% by mass, or 30% by mass to 75% by mass base on the total mass of the preparation. The starch contributes to rapid disintegrability in the oral cavity. When the starch content is 20% by mass or more or falls within the above ranges, the disintegrability in the oral cavity tends to be particularly good.

The starch may have any particle size. It should be noted that the starch may have a particle diameter of 100 $\mu$m or less when mixed with a liquid mixture of water, 95% ethanol, and glycerin (1 : 1 : 1 in volume ratio) and then observed with an optical microscope. If the starch has too large a particle size, it will tend to have a grainy feel in the oral cavity during taking. In general, starch not having undergone a pharmaceutical process is said to have not so good compression moldability. However, the orally disintegrating tablet according the embodiment can be produced using starch not having undergone a pharmaceutical process such as granulation. Rather, in such a case, more rapid disintegrability in the oral cavity can be obtained.

The specific inorganic excipient (c) that may be contained in the orally disintegrating tablet according to the embodiment is at least one inorganic excipient selected from the group consisting of magnesium silicate, calcium silicate, and synthetic hydrotalcite. Such an inorganic excipient can impart a high strength to the tablet and is less soluble in water and non-water-absorptive.

The inorganic excipient may have any particle size. It should be noted that the inorganic excipient may have an average particle size of 1 to 50 $\mu$m when measured by a laser diffraction scattering method.

The content of at least one inorganic excipient selected from the group consisting of magnesium silicate, calcium silicate, and synthetic hydrotalcite may be 3% by mass to 60% by mass, 3% by mass to 50% by mass, 4% by mass to 45% by mass, or 7% by mass to 40% by mass based on the total mass of the preparation. The inorganic excipient plays a role in imparting a high strength to the tablet. When the content of the inorganic excipient is moderately high based on the total mass of the preparation and specifically falls within the above ranges, the problem with the compression moldability of starch can be more easily overcome.

Although not essential, the orally disintegrating tablet according to the embodiment may further contain an additional inorganic excipient other than the above. The additional inorganic excipient may be such that it has a solubility of 0.1 g/L or less in water at 20 $\pm$ 5°C. Specifically, examples of the additional inorganic excipient include kaolin, hydrated silicon dioxide, amorphous silicon oxide hydrate, dried aluminum hydroxide gel, magnesium aluminosilicate, aluminum magnesium silicate, light anhydrous silicic acid, synthetic aluminum silicate, titanium oxide, magnesium oxide, magnesia alumina hydrate, aluminum hydroxide gel, aluminum hydroxide-sodium bicarbonate co-precipitate, aluminum hydroxide-magnesium hydrogen carbonate-calcium carbonate co-precipitate, magnesium hydroxide, tricalcium phosphate, calcium carbonate, precipitated calcium carbonate, magnesium carbonate, silicon dioxide, bentonite, anhydrous silicic acid hydrate, anhydrous dibasic calcium phosphate, a granulated substance of anhydrous dibasic calcium phosphate, calcium monohydrogen phosphate, dibasic calcium phosphate hydrate, calcium hydrogen phosphate granules, and the like. When the orally disintegrating tablet further contains any of these additional excipients, at least one inorganic excipient (c) selected from the group consisting of magnesium silicate, calcium silicate, and synthetic hydrotalcite may make up at least 50% by mass of the total mass of the inorganic excipients so that a preferred level of tablet strength can be obtained. If the additional inorganic excipient is used in a large amount, it may strongly adhere to a mortar or a punch and may cause a tableting machine to wear off so that foreign metallic particles may occur.

Next, the active ingredient (a) for use in the orally disintegrating tablet according to the embodiment will be described. The active ingredient to be used may have any physical properties of powder, any shape, and any features. For example, a powdery or crystalline raw material of the active ingredient may be directly mixed with a part or all of the other ingredients, depending on the purpose. The raw material may be directly used as the active ingredient or may be sieved so that the active ingredient can be used in the form of particles with a controlled size.

The orally disintegrating tablet may contain the active ingredient by itself or may contain particles containing the active ingredient (active ingredient-containing particles). The active ingredient-containing particles may be a product obtained by subjecting the active ingredient to a pharmaceutical process such as granulation using a pharmaceutically acceptable additive for the purpose of controlled release, bitter taste masking, solubilization, or the like, without departing from the gist of the present invention. Examples of the active ingredient-containing particles include granulated particles, microcapsules, and coated granules. The active ingredient-containing particles may contain an excipient identical to or different from the inorganic excipient or the like in addition to the active ingredient. The content of the active ingredient in the active ingredient-containing particles may be typically, but not limited to, 0.001% by mass to 100% by mass. The active ingredient-containing particles can be produced by a conventional method known to those skilled in the art, such as stirring granulation, fluidized bed granulation, or dry granulation, using a pharmaceutically acceptable solvent, purified water, or the like.

The active ingredient (a) for use in the orally disintegrating tablet according to the embodiment may be any orally-administrable active ingredient with any type of efficacy. Examples of the active ingredient include gastrointestinal drugs, chemotherapeutics, bronchodilators, cardiotonics, antihypertensive drugs, vasodilators, vasopressors, capillary stabilizers, antipyretic analgesic and anti-inflammatory agents, antilipemic agents, antipsychotic drugs, antibiotics, antiepileptics, antiparkinson agents, antihistaminic drugs, anti-anxiety drugs, osteoporosis drugs, skeletal muscle relaxants, sedative-hypnotics, antidiarrheals, antiulcer agents, autonomic agents, psychotropic agent, antacids, intestinal drugs, antitussive and expectorant drugs, antispasmodics, arthrifuges, antidiabetic agents, antiarrhythmic agents, hormone drugs, diuretics, and the like. Examples also include bronchodilators such as PDE inhibitors, vasodilators such as dihydropyridine calcium antagonists, and antidiabetic drugs such as $\alpha$-glucosidase inhibitors. These may be used singly or in combination of two or more. The active ingredient may also be a supplement such as vitamin or amino acid or a nutrient material.

In some embodiments, water-soluble active ingredients may be used among these active ingredients. The term "water-soluble active ingredient" means that the amount of water required to dissolve 1 g or 1 mL of the active ingredient is less than 1,000 mL at 20 ± 5°C. The amount of water required to dissolve the water-soluble active ingredient may be less than 100 mL, 30 mL, or 10 mL.

Examples of the active ingredient for which the required amount of water is less than 10 mL include central nervous system drugs such as levetiracetam, chlorpromazine hydrochloride, loxoprofen sodium, sodium valproate, selegiline hydrochloride, tiapride hydrochloride, milnacipran hydrochloride, pramipexole hydrochloride hydrate, dosulepin hydrochloride, dimetotiazine mesilate, amantadine hydrochloride, clomipramine hydrochloride, imipramine hydrochloride, ethosuximide, sultopride hydrochloride, emorfazone, taltirelin hydrate, pentazocine hydrochloride, tiaramide hydrochloride, tramadol hydrochloride, amitriptyline hydrochloride, talipexole hydrochloride, amfenac sodium hydrate, pipamperone hydrochloride, flurazepam hydrochloride, clorazepate dipotassium, methylphenidate hydrochloride, ropinirole hydrochloride and nalfurafine hydrochloride; peripheral nerve drugs such as aclatonium napadisilate, distigmine bromide, N-methylscopolamine methylsulfate, scopolamine butylbromide, propantheline bromide, pyridostigmine bromide, neostigmine bromide, eperisone hydrochloride, tolperisone hydrochloride, and ambenonium chloride; sense organ drugs such as betahistine mesilate and dl-isoprenaline hydrochloride; circulatory drugs such as etilefrine hydrochloride, aprindine hydrochloride, procainamide hydrochloride, bisoprolol fumarate, oxprenolol hydrochloride, isosorbide, betaxolol hydrochloride, metoprolol tartrate, tilisolol hydrochloride, losartan potassium, trapidil, trimetazidine hydrochloride, proxyphylline, propranolol hydrochloride, acebutolol hydrochloride, bufetolol hydrochloride, pilsicainide hydrochloride hydrate, alprenolol hydrochloride, mexiletine hydrochloride, disopyramide phosphate, quinapril hydrochloride, perindopril erbumine, celiprolol hydrochloride, benazepril hydrochloride, aliskiren fumarate, sumatriptan succinate, diltiazem hydrochloride, dextran sulfate sodium sulfur 18, pravastatin sodium, gamma-aminobutyric acid, beraprost sodium, and meclofenoxate hydrochloride; respiratory drugs such as fudosteine, L-methylcysteine hydrochloride, L-ethylcysteine hydrochloride, cloperastine hydrochloride, salbutamol sulfate, pentoxyverine citrate, terbutaline sulfate, and tulobuterol hydrochloride; digestive organ drugs such as roxatidine acetate hydrochloride, methylmethionine sulfonium chloride, ranitidine hydrochloride, rabeprazole sodium, sodium picosulfate hydrate, itopride hydrochloride, cevimeline hydrochloride hydrate, egualen sodium hydrate, pirenzepine hydrochloride anhydride, tetracycline hydrochloride, ramosetron hydrochloride, domiphen bromide, granisetron hydrochloride, pilocarpine hydrochloride, indisetron hydrochloride, cetylpyridinium chloride monohydrate, azasetron hydrochloride, and tropisetron hydrochloride; hormone drugs such as kallidinogenase, conjugated estrogen, and thiamazole; urogenital and anal drugs such as ritodrine hydrochloride, oxybutynin hydrochloride, fesoterodine fumarate, and solifenacin succinate; vitamin compounds such as fursultiamin hydrochloride, flavin adenine dinucleotide sodium, ascorbic acid, calcium pantothenate, pyridoxine hydrochloride, dicethiamine hydrochloride hydrate, and hydroxocobalamin acetate; nutrients and tonics such as potassium iodide, potassium L-aspartate, potassium aspartate, magnesium aspartate, monobasic sodium phosphate monohydrate, calcium chloride hydrate, calcium L-aspartate hydrate, glucose, potassium chloride, dried iron sulfate, and anhydrous disodium hydrogen phosphate; fluid and electrolyte replenishers such as warfarin potassium, adrenochrome monoaminoguanidine mesilate hydrate, limaprost alfadex, sodium citrate, tranexamic acid, and clopidogrel sulfate; metabolic drugs such as potassium citrate, levocarnitine chloride, anagliptin, sapropterin hydrochloride, protoporphyrin disodium, voglibose, lactitol hydrate, diisopropylamine dichloroacetate, adenosine triphosphate disodium hydrate, acarbose, lysozyme hydrochloride, L-arginine hydrochloride, L-arginine, inosine pranobex, fingolimod hydrochloride, sodium citrate hydrate, vildagliptin, L-cysteine, metformin hydrochloride, monoammonium glycyrrhizinate, glutathione, buformin hydrochloride, miglitol, etidronate disodium, tiopronin, teneligliptin hydrobromide hydrate, zinc acetate hydrate, sodium phenylbutyrate, calcium disodium edetate hydrate, miglustat, mizoribine, trientine hydrochloride, and penicillamine; anti-tumor drugs such as imatinib mesylate, estramustine phosphate sodium hydrate, cytarabine ocfosfate hydrate, hydroxycarbamide, and procarbazine hydrochloride; tissue/cell functional drugs such as suplatast tosilate, d-chlorpheniramine maleate, cetirizine hydrochloride, homochlorcyclizine hydrochloride, and promethazine hydrochloride; antibiotics such as potassium clavulanate, cefotiam hexetil hydrochloride, kanamycin monosulfate, colistin sodium methanesulfonate, clindamycin hydrochloride, vancomycin hydrochloride, cloxacillin sodium hydrate, doxycycline hydrochloride hydrate, faropenem sodium hydrate, pivmecillinam hydrochloride, lincomycin hydrochloride, hydrate, and polymyxin B sulfate; chemotherapeutics such as ethambutol hydrochloride, indinavir sulfate ethonolate, raltegravir potassium, isoniazid, emtricitabine, ribavirin, oseltamivir phosphate, isoniazid sodium methanesulfonate hydrate, valganciclovir hydrochloride, and valacyclovir hydrochloride; anti-parasitic drugs such as diethylcarbamazine citrate; diagnostic drugs such as tartaric acid and urea; alkaloidal narcotics such as cocaine hydrochloride, ethylmorphine hydrochloride hydrate, oxycodone hydrochloride hydrate, codeine phosphate hydrate, dihydrocodeine phosphate, and opium alkaloid hydrochloride; non-alkaloidal narcotics such as pethidine hydrochloride; and other drugs mainly for non-therapeutic use, such as varenicline tartrate.

Examples of the active ingredient for which the required amount of water is less than 30 mL (not less than 10 mL) include central nervous system drugs such as donepezil hydrochloride, trazodone hydrochloride, lobenzarit disodium, gabapentine, methixene hydrochloride, memantine hydrochloride, and rilmazafone hydrochloride hydrate; peripheral nerve drugs such as tizanidine hydrochloride; circulatory drugs such as aminophylline hydrate, carteolol hydrochloride, pirmenol hydrochloride hydrate, lisinopril hydrate, hydralazine hydrochloride, captopril, imidapril hydrochloride, clonidine hydrochloride, midodrine hydrochloride, rizatriptan benzoate, dilazep hydrochloride hydrate, and fluvastatin sodium; respiratory drugs such as fenoterol hydrobromide, procaterol hydrochloride hydrate, clenbuterol hydrochloride, and benproperine; digestive organ drugs such as benexate hydrochloride betadex; hormone drugs such as desmopressin acetate hydrate; urogenital and anal drugs such as propiverine hydrochloride and vardenafil hydrochloride hydrate; vitamin compounds such as cobamamide; nutrients and tonics such as calcium gluconate hydrate and calcium glycerophosphate; fluid and electrolyte replenishers such as ticlopidine hydrochloride; metabolic drugs such as risedronate sodium hydrate, pronase, sitagliptin phosphate hydrate, DL-methionine, and taurine; anti-tumor drugs such as cyclophosphamide hydrate and doxifluridine; antibiotics such as cycloserine, bacampicillin hydrochloride, and demethylchlortetracycline hydrochloride; chemotherapeutics such as lamivudine, abacavir sulfate, sanilvudine, and famciclovir; diagnostic drugs such as sodium hydrogen carbonate; alkaloidal narcotics such as morphine sulfate hydrate and morphine hydrochloride hydrate; and non-alkaloidal narcotics such as methadone hydrochloride.

Examples of the active ingredient for which the required amount of water is less than 100 mL (not less than 30 mL) include central nervous system drugs such as acetaminophen, diclofenac sodium, mosapramine hydrochloride, clocapramine hydrochloride hydrate, tetrabenazine, duloxetine hydrochloride, atomoxetine hydrochloride, zolpidem tartrate, mianserin hydrochloride, fluvoxamine maleate, piroheptine hydrochloride, nortriptyline hydrochloride, mazaticol hydrochloride hydrate, epirizole, pentobarbital calcium, lithium carbonate, pregabalin, and escitalopram oxalate; peripheral nerve drugs such as timepidium bromide, piperidolate hydrochloride, pridinol mesilate, and methocarbamol; sense organ drugs such as difenidol hydrochloride; circulatory drugs such as cibenzoline succinate, flecainide acetate, mozavaptan hydrochloride, labetalol hydrochloride, delapril hydrochloride, enalapril maleate, terazosin hydrochloride hydrate, amosulalol hydrochloride, nicorandil, verapamil hydrochloride, and amezinium metilsulfate; respiratory drugs such as sodium azulene sulfonate hydrate, dextromethorphan hydrobromide hydrate, dimemorfan phosphate, ambroxol hydrochloride, trimetoquinol hydrochloride hydrate, clofedanol hydrochloride, and eprazinone hydrochloride; digestive organ drugs such as nizatidine and cetraxate hydrochloride; urogenital and anal drugs such as tamsulosin hydrochloride and tolterodine tartrate; vitamin compounds such as cyanocobalamin, thiamine nitrate, nicotinic acid, and mecobalamin; fluid and electrolyte replenishers such as carbazochrome sodium sulfonate hydrate; metabolic drugs such as camostat mesilate, alendronate sodium hydrate, colchicine, alogliptin benzoate, pirfenidone, and calcium folinate; anti-tumor drugs such as fluorouracil and capecitabine; tissue/cell functional drugs such as olopatadine hydrochloride and bepotastine besilate; antibiotics such as cephalexin, cefaclor, ampicillin hydrate, and minocycline hydrochloride; chemotherapeutics such as flucytosine, zidovudine, linezolid, ciprofloxacin hydrochloride, tenofovir disoproxil fumarate, and didanosine; and diagnostic drugs such as metyrapone.

Examples of the active ingredient for which the required amount of water is less than 1,000 mL (not less than 100 mL) include central nervous system drugs such as trihexyphenidyl hydrochloride, biperiden hydrochloride, actarit, levodopa, carbidopa hydrate, quetiapine fumarate, maprotiline hydrochloride, sertraline hydrochloride, trimipramine maleate, tandospirone citrate, setiptiline maleate, carpipramine hydrochloride hydrate, topiramate, droxidopa, profenamine hydrochloride, paroxetine hydrochloride hydrate, perphenazine maleate, fluphenazine maleate, pergolide mesilate, and perospirone hydrochloride; peripheral nerve drugs such as mepenzolate bromide, oxapium iodide, baclofen, butropium bromide, chlorphenesin carbamate, and tiquizium bromide; sense organ drugs such as dimenhydrinate; circulatory drugs such as arotinolol hydrochloride, atenolol, disopyramide, propafenone hydrochloride, nadolol, bepridil hydrochloride hydrate, amlodipine besylate, alacepril, methyldopa hydrate, doxazosin mesilate, nicardipine hydrochloride, cilazapril hydrate, bevantolol hydrochloride, cilazapril, bunazosin hydrochloride, bamidipine hydrochloride, guana-

7

benz acetate, dihydroergotamine mesilate, eletriptan hydrobromide, isoxsuprine hydrochloride, rosuvastatin calcium, ifenprodil tartrate, dihydroergotoxine mesilate, lomerizine hydrochloride, and sildenafil citrate; respiratory drugs such as theophylline and bromhexine hydrochloride; digestive organ drugs such as loperamide hydrochloride, troxipide, cimetidine, ecabet sodium hydrate, dequalinium chloride, trimebutine maleate, and mesalazine; hormone drugs such as clomiphene citrate; urogenital and anal drugs such as flavoxate hydrochloride and methylergometrine maleate; vitamin compounds such as benfotiamine and pyridoxal phosphate hydrate; nutrients and tonics such as sodium ferrous citrate; fluid and electrolyte replenishers such as aspirin, sarpogrelate hydrochloride, and edoxaban tosilate hydrate; metabolic drugs such as mitiglinide calcium hydrate, propagermanium, and cinacalcet hydrochloride; anti-tumor drugs such as melphalan, sunitinib malate, tamoxifen citrate, temozolomide, fludarabine phosphate, ubenimex, and uracil; tissue/cell functional drugs such as azelastine hydrochloride, fexofenadine hydrochloride, ketotifen fumarate, cyproheptadine hydrochloride hydrate, and bucillamine; antibiotics such as rifampicin, amoxicillin hydrate, cefroxadine hydrate, chloramphenicol, cefcapene pivoxil hydrochloride hydrate, fosfomycin calcium hydrate, and rifabutin; chemotherapeutics such as acyclovir, moxifloxacin hydrochloride, ofloxacin, terbinafine hydrochloride, garenoxacin mesilate hydrate, fluconazole, entecavir hydrate, pyrazinamide, atazanavir sulfate, levofloxacin hydrate, and lomefloxacin hydrochloride; anti-parasitic drugs such as metronidazole and mefloquine hydrochloride; and alkaloidal narcotics such as oximethebanol.

It is generally said that an orally disintegrating tablet with both rapid disintegrability in the oral cavity and high strength is difficult to design when a water-soluble active ingredient is used for it. Conventionally, therefore, there is a trend in which an orally disintegrating tablet with good physical properties is obtained by mixing a water-soluble active ingredient with large amounts of a disintegrator and an excipient such as a binder so that the content of the water-soluble active ingredient per tablet is relatively low. According to the embodiment, however, an orally disintegrating tablet with both rapid disintegrability and high strength can be obtained even when the active ingredient is water-soluble and the content of the active ingredient is high. Thus, good physical properties can be achieved even with active ingredients that have been considered to make it difficult to design orally disintegrating tablets. This means that better physical properties can be achieved with active ingredients for which formulation design is easy.

The content of the active ingredient (a) or the active ingredient-containing particles may be 0.1% by mass to 60% by mass, 2.5% by mass to 55% by mass, or 5% by mass to 50% by mass based on the total mass of the preparation, although it depends on the powder properties. The orally disintegrating tablet of the embodiment can have excellent properties even when the content of the active ingredient or the active ingredient-containing particles is high. According to the embodiment, therefore, an excellent orally disintegrating tablet can be obtained even when the content of the active ingredient or the active ingredient-containing particles is as high as, for example, 20% by mass to 60% by mass or 25% by mass to 60% by mass. The orally disintegrating tablet may contain the active ingredient-containing particles and the active ingredient dispersed by itself. In this case, the total content of the active ingredient-containing particles and the active ingredient dispersed by itself may be set in the above numerical range.

The total content of the active ingredient (a), the specific starch (b), and the specific inorganic excipient (c) may be 80% by mass or more based on the total mass of the preparation. The addition of a large amount of an ingredient other than these, such as an excipient, tends to make it difficult to obtain an orally disintegrating tablet with rapid disintegrability in the oral cavity and high strength.

The content of crystalline cellulose in the orally disintegrating tablet or the outer layer may be 5% by mass or less, 3% by mass or less, 1% by mass or less, or 0.1% by mass or less based on the total mass of the orally disintegrating tablet or the outer layer. Crystalline cellulose is often used in conventional orally disintegrating tablets. In the orally disintegrating tablet of the embodiment, however, crystalline cellulose may not only increase the excipient content needlessly but also cause a problem such as a reduction in tablet strength in some cases. Therefore, the orally disintegrating tablet or the outer layer may be substantially free of crystalline cellulose, or the crystalline cellulose content may be set within a range that does not significantly degrade the properties of the orally disintegrating tablet. Specifically, the content of crystalline cellulose may be generally set at 5% by mass or less or the above upper limit or less based on the total mass of the preparation or the outer layer.

The orally disintegrating tablet may be substantially free of water-insoluble celluloses including crystalline cellulose mentioned above, or the content of water-insoluble celluloses in the orally disintegrating tablet or the outer layer may be 5% by mass or less, 3% by mass or less, 1% by mass or less, or 0.1% by mass or less based on the total mass of the orally disintegrating tablet or the outer layer. Examples of water-insoluble celluloses include ethyl cellulose, ethylcellulose aqueous dispersion, carboxy methyl ethyl cellulose, carmellose, carmellose calcium, croscarmellose sodium, crystalline cellulose, crystalline cellulose-carmellose sodium, crystalline cellulose (minute particles), crystalline cellulose (grains), synthetic aluminum silicate-hydroxypropyl starch-crystalline cellulose, cellulose acetate, low-substituted hydroxypropyl cellulose, lactose-crystalline cellulose spherical granules, microcrystalline cellulose, hydroxypropyl methylcellulose acetate succinate, powdered cellulose, and the like. Water-insoluble celluloses have a certain level of water-absorbing ability because of their structure. Therefore, when added in a large amount, water-insoluble cellulose tends to cause a degraded mouth feel such as a grainy feel in the oral cavity by excessively absorbing the saliva in the oral cavity.

The orally disintegrating tablet may be substantially free of a water-soluble excipient, or the content of a water-soluble excipient in the orally disintegrating tablet or the outer layer may be 15% by mass or less, 10% by mass or less, 5% by mass or less, or 1% by mass or less based on the total mass of the preparation or the outer layer. When all the excipients used are water-insoluble and less water-absorptive, there is the advantage that good disintegrability in the oral cavity can be easily obtained regardless of the physical properties of the active ingredient or the like. Examples of the water-soluble excipient include saccharides, sugar alcohols, and the like.

The content of a sugar alcohol as a water-soluble excipient in the orally disintegrating tablet or the outer layer may be 0% by mass to 15% by mass, 10% by mass or less, 5% by mass or less, or 1% by mass or less based on the total mass of the orally disintegrating tablet or the outer layer. Examples of the sugar alcohol include erythritol, D-mannitol, D-sorbitol, xylitol, maltitol, anhydrous maltose, hydrous maltose, anhydrous lactitol, hydrous lactitol, and reduced maltose syrup. Sugar alcohols have high water-solubility and low moldability. The addition of these sugar alcohols without any known pharmaceutical process such as granulation tends to make it difficult to obtain a preferred level of tablet strength or to perform appropriate tableting. The acceptable content of the sugar alcohol in the orally disintegrating tablet should be the above upper limit or less, but may also be set at a level higher than the upper limit taking into account the effect of the content of other ingredients.

The same applies to saccharides as to sugar alcohols. The total content of saccharides and sugar alcohols in the orally disintegrating tablet or the outer layer may be 0% by mass to 15% by mass, 10% by mass or less, 5% by mass or less, or 1% by mass or less based on the total mass of the orally disintegrating tablet or the outer layer. Examples of saccharides include water-soluble monosaccharides and disaccharides, such as glucose, xylose, lactose, sucrose, and maltose. The orally disintegrating tablet may contain a saccharide and/or a sugar alcohol as a sweetener or the like without departing from the gist of the present invention, for example, as long as the content falls within the above numerical range.

The orally disintegrating tablet may be substantially free of water-soluble celluloses, or the content of water-soluble celluloses in the orally disintegrating tablet or the outer layer may be 5% by mass or less or 3% by mass or less based on the total mass of the orally disintegrating tablet or the outer layer. Examples of water-soluble celluloses include hydroxypropyl cellulose, hypromellose, and the like.

Crystalline cellulose and other ingredients, of which the orally disintegrating tablet of the embodiment may be substantially free, have been described above. However, any of such ingredients may be used as long as they do not significantly affect the physical properties (disintegrability and strength) of the orally disintegrating tablet. The acceptable content of such ingredients should be based on the total mass of the mixture or outer layer in which such ingredients are mixed with the starch (b) and the inorganic excipient (c). This is because such ingredients, including crystalline cellulose, can affect the tablet properties when mixed with the starch (b) and the inorganic excipient (c). For example, in the case of a dry-coated orally disintegrating tablet (dry-coated tablet) having an inner core coated with an outer layer, the physical properties of the tablet is less likely to be affected by a high content of crystalline cellulose or other ingredients when the outer layer contains the starch (b) and the inorganic excipient (c) whereas the inner core contains crystalline cellulose or other ingredients. In such a dry-coated form, the total mass of the mixture part refers to the total mass of the outer layer. In the case of a single-layer tablet (normal tablet), the total mass of the mixture part refers to the total mass of the tablet (the total mass of the preparation or the total mass of the orally disintegrating tablet). As to crystalline cellulose, therefore, the content of crystalline cellulose in a mixture with the starch (b) and the inorganic excipient (c) may be 0% by mass to 5% by mass based on the total mass of the mixture part (outer layer). Similarly, the content of a sugar alcohol in a mixture with the starch (b) and the inorganic excipient (c) may be 0% by mass to 15% by mass based on the total mass of the mixture part (outer layer).

The dry-coated orally disintegrating tablet can be produced by, for example, the dry-coated tablet-producing method described in WO 01/98067 (specifically, the process in Fig. 1). WO 03/028706 also describes a dry-coated, fast-dissolving, disintegrating, molded product produced by the dry-coated tablet producing method in WO 01/98067. In the dry-coated, fast-dissolving, disintegrating, molded product in WO 03/028706, however, the outer layer contains an ingredient with a high level of moldability and disintegrability whereas the inner core contains an active ingredient and other ingredients with a low level of moldability. WO 2010/134540 and WO 2011/071139 also describe similar dry-coated, orally disintegrating tablets.

When the orally disintegrating tablet of the embodiment is formed, the starch (b) and the inorganic excipient (c) provide a sufficient level of moldability. According to the embodiment, therefore, a dry-coated tablet having an inner core containing the active ingredient (a) can be obtained, for example, using the dry-coated tablet producing method in WO 01/98067, even when the active ingredient (a) has low moldability. In such a dry-coated orally disintegrating tablet according to the embodiment, the inner core should contain the active ingredient (a), and the outer layer should contain the starch (b) and the inorganic excipient (c), in which the active ingredient (a) may be present only in the inner core, in other words, the inner core may contain the entire amount of the active ingredient (a). In addition, the starch (b) and the inorganic excipient (c) may be present only in the outer layer. In order to control disintegrability of the inner core, the inner core may contain a part of the starch (b) and/or the inorganic excipient (c) together with the active ingredient (a).

In the dry-coated orally disintegrating tablet, the inner core may have any size, and the outer layer may have any thickness. For example, when the active ingredient is to be present only in an inner core with low moldability, good moldability should be ensured by an outer layer containing mainly the starch (b) and the inorganic excipient (c). For this purpose, the size of the inner core should be determined based on the content of the active ingredient (a), and the outer layer should be designed to have a thickness enough to ensure the moldability of the entire product, specifically, the strength of the tablet. From these points of view, for example, the inner core may have a maximum diameter of 1 mm to 11 mm, and the outer layer may have a thickness of 0.3 mm to 10.5 mm. Although the inner core and the outer layer may be present in any ratio, the content of the inner core in the whole of the orally disintegrating tablet may be, for example, 1 to 90% by mass.

If necessary, the orally disintegrating tablet of the embodiment may further contain (d) crospovidone. The orally disintegrating tablet further containing crospovidone tends to have higher strength without losing rapid disintegrability in the oral cavity. In view of this effect, the content of crospovidone may be 0.1% by mass to 20% by mass or 5% by mass to 15% by mass based on the total mass of the preparation.

The dry-coated orally disintegrating tablet may contain the crospovidone (d) in any of the outer layer and the inner core in order to have a higher level of strength and disintegrability.

Besides the above ingredients, the orally disintegrating tablet of the embodiment may contain additives generally used as pharmaceutical additives. Examples of such additives include inorganic excipients other than the above, sweeteners, corrigents, perfumes, lubricants, plasticizers, colorants, stabilizers, antioxidants, solubilizers, disintegrators, correctives, antistatic agents, adsorbents, preservatives, moistening agents, pH adjusters, and the like. The content of these additives may be 0% by mass to 20% by mass or 0% by mass to 10% by mass based on the total mass of the preparation.

If the active ingredient is unstable to light, the outer layer of the dry-coated tablet may contain any of light absorbing materials such as various dyes or pigments for improving the light stability of the active ingredient. Examples of the light absorbing material that may be added to the outer layer include Food Red No. 2, Food Red No. 3, Feed Yellow No. 4, iron sesquioxide, yellow iron sesquioxide, black iron oxide, and the like. Refer to WO 2010/087462, which describes the light absorbing material in detail.

The orally disintegrating tablet of the embodiment has both rapid disintegrability in the oral cavity and high strength. The term "rapid disintegrability in the oral cavity" means that the disintegration time in the oral cavity (the time from when a healthy adult puts a tablet into the mouth without water to when the tablet completely disintegrates or dissolves in the mouth) is short. The oral disintegration time may be generally about 5 to about 60 seconds, about 5 to about 45 seconds, or about 5 to about 30 seconds.

The term "high strength" means that the strength of the tablet is at such a level that the tablet can be handled like conventional tablets during transportation, during preparation such as unit-dose packaging with an automatic portion packaging machine at dispensing pharmacies or the like, during use by patients, or during other situations. The strength of the tablet, which is used as an index during handling as mentioned above, can be calculated from the hardness of the tablet and the fracture area of the tablet. It is reported that tablets with a strength of 1 to 2 N/mm$^2$ can withstand PTP packaging and those with a strength of 2 N/mm$^2$ or more can be handled like conventional tablets (Yasushi Ochiai, Development of New Amlodipine OD Tablet, Shinyaku to Rinsho (Journal of New Remedies & Clinics), Vol. 58, 2009, pp. 51-57). For example, when a tablet with a fracture area of 20.0 mm$^2$ has a strength of 0.5, 1.0, 1.5, or 2.0 N/mm$^2$, its hardness is about 10.0 N, 20.0 N, 30.0 N, or 40.0 N, which means that if tablets differ by 0.5 N/mm$^2$ in strength, then they differ by about 10 N in hardness.

Concerning the size of tablets, it is said that round tablets with a diameter of 7 to 9 mm are easy to handle and swallow. An aspect may provide an orally disintegrating tablet with a diameter of 7 to 9 mm even when the content of the active ingredient or the like is high based on the total mass of the preparation. For example, the amount of the active ingredient or the like per dose can be so large as to make it necessary to form a tablet with a diameter of more than 9 mm by a conventional preparation method. In such a case, the content of the excipient may be reduced so that the content of the active ingredient or the like per tablet can be increased. This makes it possible to reduce the mass of the tablet and to reduce the diameter of the tablet to 9 mm or less. This can result in a reduction in the load on patients who take the tablet.

The orally disintegrating tablet of the embodiment can be produced by, for example, a method including the steps below. According to an embodiment, a method for producing orally disintegrating tablets includes steps of: mixing at least the active ingredient (a), the specific starch (b), and the specific inorganic excipient (c), optionally under dry conditions, to form a mixture; and then subjecting the (dry) mixture to compression molding. In the step of forming a mixture, if necessary, crospovidone (d) or the additional additive may be further mixed. In the compression molding step, the mixture may be subjected to compression molding in such a way that a desired density can be achieved.

In the step of forming a mixture, at least the starch (b) and the inorganic excipient (c) may be directly mixed without undergoing a known pharmaceutical process such as granulation. In this case, orally disintegrating tablets with both rapid disintegrability in the oral cavity and high strength can be produced by a simplified process. The mixing may be performed using a W-type mixer, a V-type mixer, a container mixer, or the like. The compression molding may be performed using a single punch tableting machine, a rotary tableting machine, or the like. The mixture may be directly subjected to compression molding (direct tableting).

The term "density" refers to the mass per volume of a tablet. The orally disintegrating tablet may have a density of 0.5 to 2.0 mg/mm$^3$, 1.0 to 1.5 mg/mm$^3$, or 1.1 to 1.3 mg/mm$^3$. To achieve such a density, those skilled in the art can select an appropriate tableting pressure from the range of 3.0 to 20.0 kN. Particularly in the above density ranges, the formulation of the embodiment makes it possible to provide orally disintegrating tablets with both rapid disintegrability in the oral cavity and high strength.

EXAMPLES

Hereinafter, the present invention will be more specifically described with reference to Examples, Comparative Examples, and Reference Examples, which however are not intended to limit the present invention at all. The tablets obtained in the Examples, the Comparative Examples, and the Reference Examples were evaluated for disintegration time, strength, and density by the test methods (1) to (3) shown below.

(1) Disintegrability (disintegration time)

The disintegration times of six tablets were measured by the disintegration test according to the Japanese Pharmacopoeia, 16th edition, and the average of the measured values was calculated. A disintegration time of 120 seconds or less was evaluated as acceptable, and more preferably, a disintegration time of 90 seconds or less was evaluated as acceptable.

(2) Strength of tablet

The hardness of each of ten tablets was measured with a tablet hardness tester (PC-30 manufactured by OKADA SEIKO CO., LTD.), and the diameter and thickness of each of ten tablets were measured with a thickness gauge (SM-528 manufactured by TECLOCK Corporation). Using the average values, the strength of the tablet was calculated from the formula below. A strength of 1.8 N/mm$^2$ or more was evaluated as acceptable, and more preferably, a strength of 2.0 N/mm$^2$ or more was evaluated as acceptable.

$$\text{Strength (N/mm}^2\text{) of tablet} = \text{average hardness (N)/fracture area (mm}^2\text{)}$$

(3) Density

The mass of each of ten tablets was measured with an electronic balance (XS-204 manufactured by Mettler-Toledo International Inc.), and the diameter and thickness of each of ten tablets were measured with a thickness gauge (SM-528 manufactured by TECLOCK Corporation). Using the average values, the density of the tablet was calculated from the formula below.

$$\text{Density (mg/mm}^3\text{)} = \text{average mass (mg)/average tablet volume (mm}^3\text{)}$$

(4) Oral disintegration test

In healthy adult men, the time was measured from when they put a tablet into their mouth without water to when the tablet completely disintegrated or dissolved in the mouth. The average of the measured values was calculated. An oral disintegration time of 45 seconds or less was evaluated as acceptable.

[Table 1]

| Content (mass%) | | Example | | Comparative Example | | | | | | | | | |
|---|---|---|---|---|---|---|---|---|---|---|---|---|---|
| | | 1 | 2 | 1 | 2 | 3 | 4 | 5 | 6 | 7 | 8 | 9 | 10 |
| Active ingredient | Miglitol | 25 | 25 | 25 | 25 | 25 | 25 | 25 | 25 | 20 | 50 | 25 | 25 |
| Starch | Corn starch | 49 | 49 | 49 | 74 | | | | | 10 | 10 | 44 | 14 |
| Inorganic excipient | Synthetic hydrotalcite | 25 | 12.5 | | | 37 | 12.5 | 12.5 | 12.5 | | | | |
| | Magnesium silicate | | | | | | | | | 41 | 23 | 10 | 10 |
| Sugar alcohol | D-mannitol | | | | | | 49 | | | | | 20 | 50 |
| | Xylitol | | | | | | | 49 | | | | | |
| | Erythritol | | | | | | | | 49 | | | | |
| Water-insoluble cellulose | Crystalline cellulose | | | | | | | | | 28 | 16 | | |
| Excipient | Crospovidone | | 12.5 | 25 | | 37 | 12.5 | 12.5 | 12.5 | | | | |
| Lubricant | Sodium stearyl fumarate | 1 | 1 | 1 | 1 | 1 | 1 | 1 | 1 | 1 | 1 | 1 | 1 |

Industrial Applicability

The tablets of the Examples and the Comparative Examples were prepared so as to have a density of 1.1 to 1.3 mg/mm$^3$ by the method described below in which the tableting pressure and other conditions were controlled.

(Example 1)

According to the proportion in Table 1, each ingredient was weighed and sieved with a sieve. The ingredients were then mixed in a V-type mixer (manufactured by TOKUJU CORPORATION). The mixture was formed into tablets each with a mass of 200 mg using a tableting machine (VIRG manufactured by KIKUSUI SEISAKUSHO LTD.). This process was performed using a round punch with a diameter of 8.5 mm. The shape of the tablets corresponds to that of the punch. Miglitol was used as an active ingredient. Miglitol is water-soluble and conventionally difficult to be formed into an orally disintegrating tablet with both rapid disintegrability in the oral cavity and high strength. The amount of water required to dissolve 1 g of miglitol is less than 10 mL. As described below, corn starch used in Example 1 has an amylose content of 25 to 30% by mass and a degree of gelatinization of less than 10%.

(Example 2)

In Example 2, tablets were prepared according to the proportion in Table 1 under the same conditions as those in Example 1, except that another excipient (crospovidone) was used in addition to the inorganic excipient (synthetic hydrotalcite) for Example 1.

(Comparative Example 1)

In Comparative Example 1, tablets were prepared according to the proportion in Table 1 under the same conditions as those in Example 1, except that the inorganic excipient for Example 1 was replaced by another excipient (crospovidone).

(Comparative Example 2)

In Comparative Example 2, tablets were prepared according to the proportion in Table 1 under the same conditions as those in Example 1, except that the inorganic excipient for Example 1 was not used.

(Comparative Example 3)

In Comparative Example 3, tablets were prepared according to the proportion in Table 1 under the same conditions as those in Example 1, except that starch for Example 1 was replaced by the inorganic excipient and another excipient (crospovidone).

(Comparative Examples 4 to 6)

In Comparative Examples 4 to 6, tablets were prepared according to the proportion in Table 1 under the same conditions as those in Example 1, except that starch for Example 2 was replaced by a sugar alcohol.

(Comparative Examples 7 and 8)

In Comparative Examples 7 and 8, tablets were prepared according to the proportion in Table 1 under the same conditions as those in Example 1, except that crystalline cellulose, which is frequently used in conventional techniques, was added to the formulation according to the present invention.

(Comparative Examples 9 and 10)

In Comparative Examples 9 and 10, tablets were prepared according to the proportion in Table 1 under the same conditions as those in Example 1, except that a sugar alcohol, which is frequently used in conventional techniques, was added to the formulation according to the present invention.

[Table 2]

| | Example | | Comparative Example | | | | | | | | | |
|---|---|---|---|---|---|---|---|---|---|---|---|---|
| | 1 | 2 | 1 | 2 | 3 | 4 | 5 | 6 | 7 | 8 | 9 | 10 |
| Disintegra tion time (seconds) | 42 | 88 | 134 | 65 | 128 | 56 | 93 | Tableting is impossible | 10.5 | 10.2 | 13.2 | 14.8 |
| Strength (N/mm$^2$) | 2.21 | 3.18 | 3.45 | 0.64 | 8.97 | 1.65 | 1.75 | | 1.16 | 0.43 | 1.04 | 0.46 |
| Density (mg/mm$^3$) | 1.18 | 1.18 | 1.15 | 1.19 | 1.17 | 1.16 | 1.17 | | 1.18 | 1.17 | 1.19 | 1.19 |
| Tableting trouble (capping) | Abs ent | Abs ent | Abse nt | Abse nt | Abse nt | Prese nt | Prese nt | Present | Abse nt | Abse nt | Abse nt | Abse nt |

Table 2 shows the results of the evaluation of the tablets of Examples 1 and 2 and Comparative Examples 1 to 10. The tablet of Example 1, including the active ingredient, the specific starch, and the specific inorganic excipient, had a strength of 2.0 N/mm$^2$ or more and a disintegration time of 90 seconds or less, which were good. The tablet of Example 2, further including crospovidone, also had a strength of 2.0 N/mm$^2$ or more and a disintegration time of 90 seconds or less, which were as good as in Example 1. The tablets of Comparative Examples 1 and 3, in which synthetic hydrotalcite or corn starch was excluded from the formulation of Example 1 and replaced by crospovidone, had a high strength of 2.0 N/mm$^2$ or more but a prolonged disintegration time. The tablet of Comparative Example 2, in which synthetic hydrotalcite was excluded from the formulation of Example 1, had an acceptable disintegration time but a strength as low as 0.64 N/mm$^2$. In Comparative Examples 4 to 6, where starch for the formulation of Example 2 was replaced by a sugar alcohol, a significant tableting trouble (capping) was observed in the tableting process, and tablets were difficult to produce in Comparative Example 6. In Comparative Examples 4 and 5, the strength of the tablets was as low as about 1.7 N/mm$^2$. In Comparative Examples 7 and 8 with a crystalline cellulose content of 10% by mass, the resulting strength of the tablets was not as desired.

From these results, it has been found that a combination of the specific starch (b) and the specific inorganic excipient (c) is particularly important for the orally disintegrating tablet and allows the tablet to have physical properties better than those obtained when one of them is replaced by a different excipient. It has also been found that when such a combination is used, the addition of an excess of crystalline cellulose can have an adverse effect on the physical properties of the orally disintegrating tablet. High tablet strengths were obtained in the Examples where the crystalline cellulose content was 0% by mass. This suggests that a crystalline cellulose content of about 5% by mass or less will not substantially affect the physical properties of the orally disintegrating tablet.

(Study 1 of starch)

A study was conducted on whether different amylose contents of starch could have different effects.

[Table 3]

| Content (mass%) | | Amylose content (mass%) | Example 3 | Example 4 | Comparative Example 11 |
|---|---|---|---|---|---|
| Active ingredient | Miglitol | - | 25 | 25 | 25 |
| Starch | Corn starch | 25 or more and less than 30 | 54 | | |
| | Potato starch | 20 or more and less than 25 | | 54 | |
| | Rice starch | 15 or more and less than 20 | | | 54 |
| Inorganic excipient | Synthetic hydrotalcite | - | 10 | 10 | 10 |
| Excipient | Crospovidone | - | 10 | 10 | 10 |
| Lubricant | Sodium stearyl fumarate | - | 1 | 1 | 1 |

(Example 3)

In Example 3, tablets were prepared with each ingredient weighed according to the proportion in Table 3, under the same conditions as those in Example 1.

(Example 4)

In Example 4, tablets were prepared according to the proportion in Table 3 under the same conditions as those in Example 3, except that corn starch for Example 3 was replaced by potato starch.

(Comparative Example 11)

In Comparative Example 11, tablets were prepared according to the proportion in Table 3 under the same conditions as those in Example 3, except that corn starch for Example 3 was replaced by rice starch.

[Table 4]

|  | Example 3 | Example 4 | Comparative Example 11 |
|---|---|---|---|
| Disintegration time (seconds) | 69 | 21 | 305 |
| Strength (N/mm$^2$) | 2.27 | 1.85 | 4.22 |
| Density (mg/mm$^3$) | 1.18 | 1.19 | 1.18 |

Table 4 shows the results of the evaluation of the tablets of Examples 3 and 4 and Comparative Example 11. The tablets of Examples 3 and 4, prepared using starch with an amylose content of 20 to 30% by mass, all had a strength of 1.8 N/mm$^2$ or more and a disintegration time of 90 seconds or less, which were good. The tablet of Comparative Example 11, prepared using starch with an amylose content of 15 to 20% by mass, had a good strength but a significantly prolonged disintegration time. This shows that high tablet strength and rapid disintegrability can be obtained using starch with an amylose content of 20 to 30% by mass.

(Study 2 of starch)

A study was conducted on whether different starch gelatinization degrees could have different effects.

[Table 5]

| Content (mass%) | | Gelatinization degree (%) | Example 5 | Comparative Example 12 | Comparative Example 13 |
|---|---|---|---|---|---|
| Active ingredient | Miglitol | - | 25 | 25 | 25 |
| Starch | Corn starch | Less than 10 | 59 | | |
| | Partially gelatinized starch | 55 to 70 | | 64 | |
| | Gelatinized starch | 90 to 100 | | | 64 |
| Inorganic excipient | Synthetic hydrotalcite | - | 15 | 10 | 10 |
| Lubricant | Sodium stearyl fumarate | - | 1 | 1 | 1 |

(Example 5)

In Example 5, tablets were prepared with each ingredient weighed according to the proportion in Table 5 under the same conditions as those in Example 1.

(Comparative Examples 12 and 13)

In Comparative Examples 12 and 13, tablets were prepared according to the proportion in Table 5 under the same conditions as those in Example 5, except that corn starch for Example 5 was replaced by partially gelatinized starch or gelatinized starch.

[Table 6]

|  | Example 5 | Comparative Example 12 | Comparative Example 13 |
|---|---|---|---|
| Disintegration time (seconds) | 47 | 606 | 1800 or more |
| Strength (N/mm$^2$) | 2.13 | 1.09 | 2.09 |
| Density (mg/mm$^3$) | 1.21 | 1.16 | 1.13 |

Table 6 shows the results of the evaluation of the tablets of Example 5 and Comparative Examples 12 and 13. It has been found that as the gelatinization degree of starch increases, the disintegration time significantly increases. When the gelatinization degree of starch was high, a significant increase in pressure was observed in the tableting process even though the tableting was performed under the same conditions. This shows that rapid disintegrability in the oral cavity can be obtained using starch with a degree of gelatinization of less than 10%.

(Study 1 of inorganic excipient)

A study was conducted on whether different types of inorganic excipients could have different effects.

[Table 7]

| Content (mass%) | | Example 6 | Example 7 | Example 8 | Comparative Example 14 |
|---|---|---|---|---|---|
| Active ingredient | Miglitol | 25 | 25 | 25 | 25 |
| Starch | Corn starch | 54 | 54 | 54 | 54 |
| Inorganic excipient | Synthetic hydrotalcite | 10 | | | |
| | Magnesium silicate | | 10 | | |
| | Calcium silicate | | | 10 | |
| | Anhydrous calcium hydrogen phosphate | | | | 10 |
| Excipient | Crospovidone | 10 | 10 | 10 | 10 |
| Sweetener | Sucralose | 0.05 | 0.05 | 0.05 | |
| Lubricant | Sodium stearyl fumarate | 1 | 1 | 1 | 1 |

(Example 6)

In Example 6, tablets were prepared with each ingredient weighed according to the proportion in Table 7 under the same conditions as those in Example 1.

(Example 7)

In Example 7, tablets were prepared according to the proportion in Table 7 under the same conditions as those in Example 6, except that synthetic hydrotalcite as the inorganic excipient for Example 6 was replaced by magnesium silicate.

(Example 8)

In Example 8, tablets were prepared according to the proportion in Table 7 under the same conditions as those in Example 6, except that synthetic hydrotalcite as the inorganic excipient for Example 6 was replaced by calcium silicate.

(Comparative Example 14)

In Example 14, tablets were prepared according to the proportion in Table 7 under the same conditions as those in Example 6, except that synthetic hydrotalcite as the inorganic excipient for Example 6 was replaced by anhydrous calcium hydrogen phosphate.

[Table 8]

| | Example 6 | Example 7 | Example 8 | Comparative Example 14 |
|---|---|---|---|---|
| Disintegration time (seconds) | 78 | 53 | 53 | 43 |
| Strength (N/mm$^2$) | 2.86 | 2.61 | 3.76 | 1.45 |

(continued)

|  | Example 6 | Example 7 | Example 8 | Comparative Example 14 |
|---|---|---|---|---|
| Density (mg/mm$^3$) | 1.18 | 1.18 | 1.17 | 1.17 |

Table 8 shows the results of the evaluation of the tablets of Examples 6 to 8 and Comparative Example 14. The tablets of Examples 6 to 8, prepared with synthetic hydrotalcite, magnesium silicate, or calcium silicate as the inorganic excipient, had a strength of 2.0 N/mm$^2$ or more and a disintegration time of 90 seconds or less, which were good properties. The tablet of Comparative Example 14, prepared with anhydrous calcium hydrogen phosphate as the inorganic excipient, had an acceptable disintegration time but a strength as low as 1.45 N/mm$^2$. This shows that among inorganic excipients, synthetic hydrotalcite, magnesium silicate, and calcium silicate are particularly preferred.

(Study 2 of inorganic excipient)

A study was conducted on whether different inorganic excipient contents could have different effects.

[Table 9]

| Content (mass%) | | Example 5 | Example 9 | Example 10 | Reference Example 1 | Reference Example 2 |
|---|---|---|---|---|---|---|
| Active ingredient | Miglitol | 25 | 25 | 25 | | |
| Starch | Corn starch | 59 | 39 | 34 | 94 | 89 |
| Inorganic excipient | Synthetic hydrotalcite | 15 | 35 | 40 | 5 | 10 |
| Lubricant | Sodium stearyl fumarate | 1 | 1 | 1 | 1 | 1 |

(Example 5)

In Example 5, tablets were prepared with each component weighed according to the proportion in Table 9 as mentioned above, under the same conditions as those in Example 1.

(Examples 9 and 10)

In Examples 9 and 10, tablets were prepared according to the proportion in Table 9 under the same conditions as those in Example 5, except that the content of the inorganic excipient was different from that in Example 5.

(Reference Examples 1 and 2)

In Reference Examples 1 and 2, tablets were prepared according to the proportion in Table 9 under the same conditions as those in Example 5, except that the active ingredient and a part of synthetic hydrotalcite for Example 5 were replaced by corn starch. In Reference Examples 1 and 2, the content of the active ingredient was reduced to eliminate the effect of the active ingredient as much as possible so that the effective lower limit of synthetic hydrotalcite could be found. Therefore, Reference Examples 1 and 2 may be regarded as Examples with a very low content of the active ingredient.

[Table 10]

|  | Example 5 | Example 9 | Example 10 | Reference Example 1 | Reference Example 2 |
|---|---|---|---|---|---|
| Inorganic excipient content (mass%) | 15 | 35 | 40 | 5 | 10 |
| Disintegration time (seconds) | 47 | 38 | 36 | 42 | 29 |

(continued)

|  | Example 5 | Example 9 | Example 10 | Reference Example 1 | Reference Example 2 |
|---|---|---|---|---|---|
| Strength (N/mm$^2$) | 2.13 | 2.85 | 3.15 | 2.13 | 2.13 |
| Density (mg/mm$^3$) | 1.21 | 1.18 | 1.17 | 1.16 | 1.15 |

Table 10 shows the results of the evaluation of the tablets. The tablets of Examples 5, 9, and 10 with an inorganic excipient content of 15 to 40% by mass had a strength of 2.0 N/mm$^2$ or more and a disintegration time of 90 seconds or less, which were good physical properties. The tablets of Reference Examples 1 and 2 with inorganic excipient contents of 5% by mass and 10% by mass, respectively, also had a strength of 2.0 N/mm$^2$ or more and a disintegration time of 90 seconds or less, which were as good physical properties as in Example 1. These results indicate that tablets with an inorganic excipient content of about 3% by mass or more can have desired physical properties.

The range of the inorganic excipient content will be discussed. It is suggested that the specific properties of the orally disintegrating tablet may still remain even if the active ingredient miglitol is removed from the formulation of Example 10 in which the inorganic excipient content is 40% by mass. It is therefore suggested that tablets with particularly good physical properties can be obtained when the inorganic excipient content is about 60% by mass or less, 55% by mass or less, or 50% by mass or less. The results on Reference Examples 1 and 2 suggest that in order for the orally disintegrating tablet to have specific properties, the presence of an active ingredient is not always essential. This suggests that a dry-coated orally disintegrating tablet can also have good physical properties when composed of an active ingredient-containing inner core and an outer layer containing the specific starch and the specific inorganic excipient.

(Study of starch and active ingredient)

A study was conducted on whether different starch contents and different active ingredient contents could have different effects.

[Table 11]

| Content (mass%) | | Example 2 | Example 11 | Example 12 | Reference Example 1 |
|---|---|---|---|---|---|
| Active ingredient | Miglitol | 25 | 5 | 50 | |
| Starch | Corn starch | 49 | 69 | 24 | 94 |
| Inorganic excipient | Synthetic hydrotalcite | 12.5 | 12.5 | 12.5 | 5 |
| Excipient | Crospovidone | 12.5 | 12.5 | 12.5 | |
| Lubricant | Sodium stearyl fumarate | 1 | 1 | 1 | 1 |

(Example 2)

In Example 2, tablets were prepared with each ingredient weighed according to the proportion in Table 11 as mentioned above, under the same conditions as those in Example 1.

(Examples 11 and 12)

In Examples 11 and 12, tablets were prepared according to the proportion in Table 11 under the same conditions as those in Example 2, except that the contents of the active ingredient and starch for Example 2 were changed.

(Reference Example 1)

In Reference Example 1, tablets were prepared according to the proportion in Table 11 under the same conditions as those in Example 2. This example may be regarded as a modification of Example 2, in which the active ingredient, crospovidone, and a part of synthetic hydrotalcite for Example 2 were replaced by corn starch.

[Table 12]

|  | Example 2 | Example 11 | Example 12 | Reference Example 1 |
|---|---|---|---|---|
| Active ingredient content (mass%) | 25 | 5 | 50 | 0 |
| Starch content (mass%) | 49 | 69 | 24 | 94 |
| Disintegration time (seconds) | 88 | 45 | 105 | 42 |
| Strength (N/mm$^2$) | 3.18 | 4.90 | 2.19 | 2.13 |
| Density (mg/mm$^3$) | 1.18 | 1.19 | 1.17 | 1.16 |

Table 12 shows the results of the evaluation of the tablets of Examples 2, 11, and 12 and Reference Example 1. The tablets of Examples 2 and 11 with an active ingredient content of 5 to 25% by mass and a starch content of 49 to 69% by mass had a strength of 2.0 N/mm$^2$ or more and a disintegration time of 90 seconds or less, which were good. The tablet of Example 12 with an active ingredient content of 50% by mass and a starch content of 24% by mass had a good strength of 2.0 N/mm$^2$ or more and a disintegration time of 120 seconds or less, which was in the permissible range. The tablet of Reference Example 1 with an active ingredient content of 0% by mass and a starch content of 94% by mass had a strength of 2.0 N/mm$^2$ or more and a disintegration time of 90 seconds or less, which were good. This suggests that the orally disintegrating tablet can have particularly good physical properties when its active ingredient content is at most 60% by mass based on the total mass of the preparation and its starch content is 20 to 95% by mass depending on the active ingredient content.

(Study of other active ingredients)

A study was conducted using different types of active ingredients.

[Table 13]

| Content (mass%) |  | Example 13 | Example 14 | Example 15 |
|---|---|---|---|---|
| Active ingredient | Theophylline | 25 |  |  |
|  | Nifedipine |  | 5 |  |
|  | Coated granules |  |  | 30 |
| Starch | Corn starch | 49 | 69 | 44 |
| Inorganic excipient | Synthetic hydrotalcite | 12.5 | 12.5 | 12.5 |
| Excipient | Crospovidone | 12.5 | 12.5 | 12.5 |
| Lubricant | Sodium stearyl fumarate | 1 | 1 | 1 |

(Example 13)

In Example 13, tablets were prepared according to the proportion in Table 13 under the same conditions as those in Example 1, except that the active ingredient was replaced by theophylline. The amount of water required to dissolve 1 g of theophylline is less than 1,000 mL.

(Example 14)

In Example 14, tablets were prepared according to the proportion in Table 13 under the same conditions as those in Example 1, except that the active ingredient was replaced by nifedipine. The amount of water required to dissolve 1 g of nifedipine is at least 10,000 mL.

(Example 15)

A coating liquid was sprayed to 26.3% by mass of crystalline cellulose particles (Celphere CP-203 (trade name) manufactured by Asahi Kasei Chemicals Corporation) as core particles in a fluidized bed granulating-drying-coating machine FLO-5 (manufactured by Freund Corporation). The coating liquid was composed of 5.3% by mass of triethyl citrate (Citroflex (trade name) manufactured by MORIMURA BROS., INC.), 15.8% by mass of talc (Japanese Pharmacopoeia Talc (trade name) manufactured by Muramatsu Sangyo Co., Ltd.), and 52.6% by mass (solid basis) of methacrylic acid copolymer LD (EUDRAGIT L30D-55 (trade name) manufactured by Evonik Industries AG). The product was then dried. In Example 15, tablets were prepared using the resulting coated granules according to the proportion in Table 13, under the same conditions as those in Example 1. Although the tablet of Example 15 contains no active ingredient, the coated granules may be regarded as active ingredient-containing particles because they are obtained by subjecting crystalline cellulose particles, as a virtual active ingredient, to a pharmaceutical process for purposes such as controlled release and bitter taste masking.

[Table 14]

|  | Example 13 | Example 14 | Example 15 |
|---|---|---|---|
| Disintegration time (seconds) | 37 | 41 | 32 |
| Strength (N/mm$^2$) | 5.11 | 4.64 | 3.39 |
| Density (mg/mm$^3$) | 1.18 | 1.16 | 1.17 |

Table 14 shows the results of the evaluation of the tablets of Examples 13 to 15. The tablets of Examples 13 and 14, prepared with theophylline or nifedipine as the active ingredient, had a strength of 2.0 N/mm$^2$ or more and a disintegration time of 90 seconds or less, which were good physical properties. The tablet of Example 15, prepared with coated crystalline cellulose particles (coated granules) as an example of active ingredient-containing particles, had a strength of 2.0 N/mm$^2$ or more and a disintegration time of 90 seconds or less, which were good physical properties. These results show that a combination of the specific starch and the specific inorganic excipient can not only form tablets with good physical properties when used together with a water-soluble active ingredient, which is generally considered to be difficult to use in designing orally disintegrating tablets, but also form tablets with better physical properties when used together with a water-insoluble active ingredient. Therefore, the orally disintegrating tablet of the present invention would exhibit good physical properties regardless of the nature of the active ingredient or the active ingredient-containing granules.

(Study of dry-coated orally disintegrating tablet)

On the basis of the discussion about Reference Examples 1 and 2 above, a study was conducted on whether dry-coated orally disintegrating tablets could be actually formed.

[Table 15]

| Content (mass%) | | | Example 16 | Example 17 | Example 18 |
|---|---|---|---|---|---|
| Inner core | Active ingredient | Anhydrous caffeine | 25 | 25 | 25 |
| | Starch | Corn starch | 9.75 | 9.75 | 9.75 |
| | Lubricant | Magnesium stearate | 0.25 | 0.25 | 0.25 |
| Outer layer | Starch | Corn starch | 78.5 | 78.5 | 69.75 |
| | Inorganic excipient | Magnesium silicate | 8.75 | | 8.75 |
| | | Calcium silicate | | 8.75 | |
| | Excipient | Crospovidone | | | 8.75 |
| | Lubricant | Magnesium stearate | 0.25 | 0.25 | 0.25 |

(Example 16)

According to the proportion in Table 16, the inner core ingredients were weighed and then mixed to form a powder for the inner core. According to the proportion in Table 16, the outer layer ingredients were also weighed and then mixed to form a powder for the outer layer. Subsequently, using a round compressible punch having a double structure with an inner diameter of 6.0 mm and an outer diameter of 8.0 mm, the powders were charged in such a ratio that a composite of 20% by mass of a first outer layer, 12.5% by mass of an inner core, and 67.5% by mass of a second outer layer could be formed, and then temporarily compressed by the dry-coated tablet producing method shown in Fig.

1 of WO 01/98067. Using Autograph (AG-1 manufactured by Shimadzu Corporation), the compressed product was finally tableted under a compression pressure of about 10 kN/tablet to form dry-coated orally disintegrating tablets each with a mass of 200 mg. The contents of the inner core and the outer layer are 12.5% by mass and 87.5% by mass, respectively.

(Example 17)

In Example 17, tablets were prepared according to the proportion in Table 16 under the same conditions as those in Example 16, except that the inorganic excipient as an outer layer ingredient for Example 16 was replaced by calcium silicate.

(Example 18)

In Example 18, tablets were prepared according to the proportion in Table 16 under the same conditions as those in Example 16, except that starch as an outer layer ingredient for Example 16 was partially replaced by another excipient (crospovidone).

[Table 16]

|  | Example 16 | Example 17 | Example 18 |
|---|---|---|---|
| Disintegration time (seconds) | 41 | 30 | 36 |
| Strength (N/mm$^2$) | 2.8 | 3.11 | 3.02 |
| Density (mg/mm$^3$) | 1.21 | 1.19 | 1.18 |

Table 17 shows the results of the evaluation of the tablets of Examples 16 to 18. The dry-coated orally disintegrating tablets of Examples 16 to 18, including the active ingredient, the starch, and the inorganic excipient, had a strength of 2.0 N/mm$^2$ or more and a disintegration time of 90 seconds or less, which were good. The tablet of Example 18, containing crospovidone substituted for a part of starch as an outer layer ingredient, also had a strength of 2.0 N/mm$^2$ or more and a disintegration time of 90 seconds or less, which were as good physical properties as those in Examples 16 and 17. These results show that a combination of the specific starch and the specific inorganic excipient, which can form orally disintegrating tablets with specific properties according to the present invention, can also form dry-coated orally disintegrating tablets with good physical properties when the specific starch and the specific inorganic excipient are used to form the outer layer on the inner core containing the whole amount of the active ingredient or the active ingredient-containing particles. A mixture of anhydrous caffeine, corn starch, and magnesium stearate, used as an inner core-forming powder in Examples 16 to 18, is a much less moldable powder. These Examples exactly correspond to the dry-coated orally disintegrating tablets described in WO 03/028706, WO 2010/134540, and WO 2011/071139, in which the inner core is made of an incompletely molded product or a less moldable powder.

(Correlation between disintegration test according to the Japanese Pharmacopoeia, 16th edition and oral disintegration test)

The tablets of some of the Examples and the Comparative Examples were measured for disintegration time in the oral cavity of six healthy adult men (25 to 39 years old).

[Table 17]

|  | Example 1 | Example 2 | Example 11 | Example 12 | Comparative Example 1 | Comparative Example 2 | Comparative Example 3 |
|---|---|---|---|---|---|---|---|
| Disintegration time (seconds) | 42 | 88 | 45 | 105 | 134 | 65 | 128 |
| Oral disintegration time (seconds) | 24.0 | 24.0 | 8.5 | 43.0 | 50.5 | 18.5 | 47.3 |

Table 15 shows the results of the evaluation of the tablets of Examples 1, 2, 11, and 12 and Comparative Examples of 1 to 3. The tablets of Examples 1, 2, 11, and 12 and Comparative Example 2 had an oral disintegration time of at most 45 seconds, which was a good result, while they had a disintegration time of 120 seconds or less as measured by the disintegration test according to the Japanese Pharmacopoeia, 16th edition. On the other hand, the tablets of Comparative Examples 1 and 3 had an oral disintegration time of 45 seconds or more while they had a disintegration time of 120 seconds or more as measured by the Japanese Pharmacopoeia method. This indicates that there is a correlation between the fact that tablets have a disintegration time of at most 120 seconds as measured by the disintegration test according to the Japanese Pharmacopoeia, 16th edition and the fact that tablets have an oral disintegration time of at most 45 seconds.

**Reference Signs List**

1 orally disintegrating tablet, 10 mixture layer, 11 inner core, 20 outer layer.

**Claims**

1. An orally disintegrating tablet comprising:

   (a) an active ingredient;
   (b) a starch with an amylose content of 20% by mass to 30% by mass and a degree of gelatinization of less than 10%; and
   (c) at least one inorganic excipient selected from the group consisting of magnesium silicate, calcium silicate, and synthetic hydrotalcite,

   the orally disintegrating tablet being a single-layer tablet in which the active ingredient (a), the starch (b), and the inorganic excipient (c) are dispersed and mixed over the whole of the orally disintegrating tablet, the orally disintegrating tablet having a crystalline cellulose content of 0% by mass to 5% by mass based on the total mass of the orally disintegrating tablet.

2. The orally disintegrating tablet according to claim 1, which has a sugar alcohol content of 0% by mass to 15% by mass based on the total mass of the orally disintegrating tablet.

3. An orally disintegrating tablet comprising:

   (a) an active ingredient;
   (b) a starch with an amylose content of 20% by mass to 30% by mass and a degree of gelatinization of less than 10%; and
   (c) at least one inorganic excipient selected from the group consisting of magnesium silicate, calcium silicate, and synthetic hydrotalcite,

   the orally disintegrating tablet being a dry-coated tablet having an inner core and an outer layer, wherein the inner core contains the active ingredient (a), and the outer layer contains the starch (b) and the inorganic excipient (c) and has a crystalline cellulose content of 0% by mass to 5% by mass based on the total mass of the outer layer.

4. The orally disintegrating tablet according to claim 3, wherein the outer layer has a sugar alcohol content of 0% by mass to 15% by mass based on the total mass of the outer layer.

5. The orally disintegrating tablet according to any one of claims 1 to 4, which has a total content of the active ingredient (a), the starch (b), and the inorganic excipient (c) of at least 80% by mass based on the total mass of the orally disintegrating tablet.

6. The orally disintegrating tablet according to any one of claims 1 to 5, which has a content of the active ingredient (a) of 0.1% by mass to 60% by mass based on the total mass of the orally disintegrating tablet, has a content of the starch (b) of 20% by mass to 96% by mass based on the total mass of the orally disintegrating tablet, and has a content of the inorganic excipient (c) of 3% by mass to 60% by mass based on the total mass of the orally disintegrating tablet.

**7.** The orally disintegrating tablet according to claim 6, which has a content of the active ingredient (a) of 2.5% by mass to 55% by mass based on the total mass of the orally disintegrating tablet.

**8.** The orally disintegrating tablet according to claim 6, which has a content of the starch (b) of 25% by mass to 85% by mass based on the total mass of the orally disintegrating tablet.

**9.** The orally disintegrating tablet according to claim 6, which has a content of the inorganic excipient (c) of 4% by mass to 45% by mass based on the total mass of the orally disintegrating tablet.

**10.** The orally disintegrating tablet according to any one of claims 1 to 9, wherein the starch (b) comprises at least one selected from the group consisting of corn starch, potato starch, and wheat starch.

**11.** The orally disintegrating tablet according to any one of claims 1 to 10, further comprising (d) crospovidone.

**12.** The orally disintegrating tablet according to claim 11, which has a content of the crospovidone (d) of 0.1% by mass to 20% by mass based on the total mass of the orally disintegrating tablet.

**13.** The orally disintegrating tablet according to any one of claims 1 to 12, wherein the active ingredient (a) comprises a water-soluble active ingredient.

**14.** The orally disintegrating tablet according to claim 13, wherein the water-soluble active ingredient comprises an $\alpha$-glucosidase inhibitor.

**15.** The orally disintegrating tablet according to claim 14, wherein the $\alpha$-glucosidase inhibitor comprises miglitol.

Fig.1

Fig.2

## INTERNATIONAL SEARCH REPORT

| International application No. |
| --- |
| PCT/JP2014/069024 |

### A. CLASSIFICATION OF SUBJECT MATTER

*A61K47/36*(2006.01)i, *A61K9/20*(2006.01)i, *A61K31/445*(2006.01)i, *A61K47/02*(2006.01)i, *A61K47/32*(2006.01)i, *A61P3/10*(2006.01)i, *A61P43/00*(2006.01)i

According to International Patent Classification (IPC) or to both national classification and IPC

### B. FIELDS SEARCHED

Minimum documentation searched (classification system followed by classification symbols)
A61K47/36, A61K9/20, A61K31/445, A61K47/02, A61K47/32, A61P3/10, A61P43/00

Documentation searched other than minimum documentation to the extent that such documents are included in the fields searched
Jitsuyo Shinan Koho          1922–1996   Jitsuyo Shinan Toroku Koho   1996–2014
Kokai Jitsuyo Shinan Koho    1971–2014   Toroku Jitsuyo Shinan Koho   1994–2014

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2005-187349 A  (Lion Corp.), 14 July 2005 (14.07.2005), claims; paragraphs [0012] to [0024]; examples 1, 4; comparative examples 1 to 3 (Family: none) | 1,2,5-15 |
| X | WO 2011/019046 A1  (Fuji Chemical Industry Co., Ltd.), 17 February 2011 (17.02.2011), claims; example 12 & US 2012/0165413 A1    & EP 2465540 A1 | 1,5-15 |

☒ Further documents are listed in the continuation of Box C.          ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 02 October, 2014 (02.10.14) | 14 October, 2014 (14.10.14) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2014/069024

C (Continuation). DOCUMENTS CONSIDERED TO BE RELEVANT

| Category* | Citation of document, with indication, where appropriate, of the relevant passages | Relevant to claim No. |
|---|---|---|
| A | WO 2010/134540 A1 (Dainippon Sumitomo Pharma Co., Ltd.), 25 November 2010 (25.11.2010), entire text & US 2012/0064162 A1 & EP 2433652 A1 & CN 102458475 A & KR 10-2012-0034643 A | 1-15 |
| A | WO 2012/001977 A1 (Fuji Chemical Industry Co., Ltd.), 05 January 2012 (05.01.2012), entire text (Family: none) | 1-15 |
| A | JP 2010-540588 A (Laboratorios Lesvi, S.L.), 24 December 2010 (24.12.2010), entire text & US 2010/0297031 A1 & EP 2572705 A1 & WO 2009/043844 A2 & CA 2703501 A & KR 10-2010-0077187 A | 1-15 |
| A | WO 2010/134574 A1 (Dainippon Sumitomo Pharma Co., Ltd.), 25 November 2010 (25.11.2010), entire text (Family: none) | 1-15 |
| A | JP 2012-197245 A (Kowa Co., Ltd.), 18 October 2012 (18.10.2012), entire text (Family: none) | 1-15 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4162405 B
- JP 3996626 B
- JP 2010155865 A
- JP 4446177 B
- JP 5062871 B
- JP 4551627 B
- JP 5062872 B
- JP 2011513194 A
- WO 2010134574 A
- WO 2009066773 A
- JP 4802436 B
- JP 2010540588 A
- JP 2009532343 A
- JP 2011506279 A
- JP 2005507397 A
- WO 0198067 A
- WO 03028706 A
- WO 2010134540 A
- WO 2011071139 A
- WO 2010087462 A

### Non-patent literature cited in the description

- Kaitei Iyakuhin Tenkabutsu Handbook. YAKUJI NIP-PO LIMITED, 2007, 603-610
- **YASUSHI OCHIAI.** Development of New Amlodipine OD Tablet. *Shinyaku to Rinsho,* 2009, vol. 58, 51-57